(19) Europäisches Patentamt

European Patent Office

Office européen des brevets

(11) EP 0 731 070 B1

## (12) EUROPÄISCHE PATENTSCHRIFT

(45) Veröffentlichungstag und Bekanntmachung des Hinweises auf die Patenterteilung:
**19.11.1997 Patentblatt 1997/47**

(51) Int. Cl.$^6$: **C07C 2/38**, C10G 50/02

(21) Anmeldenummer: **96100625.1**

(22) Anmeldetag: **18.01.1996**

(54) **Verfahren zur Herstellung eines Oligomer-Gemisches aus alpha-omega-Diolefinen**

Process for the preparation of a mixture of oligomers from alpha, omega-diolefins

Procédé pour la préparation d'un mélange d'oligomères à partir d'alpha, oméga-oléfines

(84) Benannte Vertragsstaaten:
**BE CH DE FR GB IT LI NL SE**

(30) Priorität: **08.03.1995 DE 19508088**

(43) Veröffentlichungstag der Anmeldung:
**11.09.1996 Patentblatt 1996/37**

(73) Patentinhaber:
**HÜLS AKTIENGESELLSCHAFT**
**45764 Marl (DE)**

(72) Erfinder:
- **Fiolitakis, Emmanuel, Dr.**
  **D.48249 Dülmen (DE)**
- **Wey, Hans G., Dr.**
  **D-45470 Mülheim/Ruhr (DE)**
- **Monkiewicz, Jaroslaw, Dr.**
  **D-79618 Rheinfelden (DE)**

(56) Entgegenhaltungen:
**DE-A- 4 119 332**

**Beschreibung**

Die Erfindung betrifft ein Verfahren zur Herstellung eines Oligomer-Gemisches aus $\alpha,\omega$-Diolefinen in Gegenwart von aluminiumorganischen Verbindungen als Katalysator in flüssiger Phase sowie die bevorzugte Verwendung der nach diesem Verfahren hergestellten Stoffe.

Die Erfindung bezweckt, ein Verfahren anzugeben, mit dem eine verbesserte Produktzusammensetzung und damit eine verbesserte Wirtschaftlichkeit in der Herstellung erreicht wird.

Aus DE-OS 41 19 332 ist ein Verfahren zur Herstellung von $\alpha,\omega$-ungesättigten Oligomeren aus $\alpha,\omega$-Diolefinen bekannt. Die $\alpha,\omega$-Diolefine werden dabei in flüssiger Phase bei einer Temperatur zwischen 150 °C und 350 °C unter Zusatz von katalytisch wirkenden Mengen an aluminiumorganischen Verbindungen der allgemeinen Formel $AlX_3$ oder $AlX_2H$ zur Reaktion gebracht. Es entstehen Methylidengruppn enthaltende $\alpha,\omega$-ungesättigte Oligomere, aus denen sich gemäß DE-OS 43 00 418 biologisch abbaubare Grundöle für Schmierstoffe und funktionelle Flüssigkeiten gewinnen lassen. Desweiteren sind aus den Oligomeren wertvolle Rohprodukte für den Lack- und Klebstoffsektor herstellbar.

Das Verfahren nach DE-OS 41 19 332 liefert bei der Herstellung der Oligomeren eine breite Produktverteilung, wobei über 70 % der Oligomere einen Oligomerisierungsgrad im Bereich von 3 bis 20 besitzen. Eine Angabe über die Art der Verteilung wird nicht gemacht. Die Produktausbeute an Oligomeren insgesamt schwankt deutlich und beträgt zwischen ca. 40 % und 85 % bezogen auf die molare Menge an eingesetztem Diolefin.

Zur Verwendung in Schmierölsektor sind jedoch wegen der besonderen Anforderungen hinsichtlich der Verdampfungsverluste (geringer Gehalt an Leichtsiedern) und der Viskosität (keine zu hohen Oligomerisierungsgrade) vor allem die Tetra-, Penta- und Hexamere der eingesetzten $\alpha,\omega$-Diolefine von Interesse.

Aufgabe der vorliegenden Erfindung ist es daher, ein Verfahren zur Herstellung eines Oligomer-Gemisches aus $\alpha,\omega$-Diolefinen anzugeben, das eine enge Produktverteilung um den Oligomerisierungsgradbereich 4 bis 5 aufweist und damit zu verbesserten Produkteigenschaften führt.

Diese Aufgabe wird erfindungsgemäß gelöst durch ein Verfahren zur Herstellung eines Oligomer-Gemisches in flüssiger Phase aus $\alpha,\omega$-Diolefinen in Gegenwart von aluminiumorganischen Verbindungen als Katalysator nach Patentanspruch 1, das sich - zumindest gedanklich - in zumindest zwei zeitlich aufeinanderfolgende Reaktionsabschnitte oder -durchläufe unterteilen läßt. Das Reaktionsprodukt jedes dieser Durchläufe wird dabei mit Hilfe eines herkömmlichen Trennverfahrens (bevorzugt destillativ) in einen ersten Teil, der zumindest den größten Teil (bevorzugt mehr als 95 Gew.-% des Gesamtgehalts) der im Rohprodukt noch enthaltenen monomeren $\alpha,\omega$-Diolefine, ihre Dimere sowie den großen Teil (bevorzugt mehr als 60 Gew.-% des Gesamtgehalts) ihrer Trimere enthält, und zumindest einen zweiten Teil, der zumindest die Tetramere und höheren Oligomere möglichst vollständig enthält, getrennt. Im allgemeinen geht dieser Trennung eine hydrolytische Zerstörung des Katalysators voraus. Besonders bevorzugt wird als Trennverfahren die Kurzweg-Vakuum-Destillation eingesetzt, um die thermische Belastung des Rohprodukts eines Durchlaufs gering zu halten.

Aus dem so erhaltenen ersten, die Leichtsieder enthaltenden Teil wird bevorzugt ein Teil der darin enthaltenen Monomere und Dimere abgetrennt und aus des Verfahren ausgeschleust, und zwar vorzugsweise ein Anteil von 20 bis 40 Gew.-% vom die Leichtsieder enthaltenden ersten Teil. Der Rest dient zusammen mit frischem Monomer und ggf. zusätzlichen anderen Olefinen als Edukt für den sich jeweils anschließenden Reaktionsdurchlauf. Dabei wird stets soviel frisches Monomer zugeführt, daß die Summe der endständigen Doppelbindungen im Eduktgemisch (nahezu) konstant bleibt. Um die durch das Recyclat (also den rückgeführten Teil an Leichtsiedern) verminderte Reaktivität des Reaktionsgemisches zu kompensieren, wird zudem von Durchlauf zu Durchlauf die Konzentration des eingesetzten Katalysators um jeweils bis zu 20 Gew.-% bezogen auf die Katalysatormasse im vorangegangenen Durchlauf erhöht, bevorzugt um ca. 12 Gew.-%. Im ersten Durchlauf beträgt die Katalysatorkonzentration bevorzugt zwischen 2 und 4 Mol.-% bezogen auf die eingesetzte Substanzmenge an $\alpha,\omega$-Diolefinen.

Zum Erreichen der gewünschten Produktzusammensetzung mit einer engen Produktverteilung um den Oligomerisierungsgradbereich 4 bis 5 ist es erfindungsgemäß erforderlich, daß die Reaktionskennzahl

Katalysator-Konzentration * Reaktionszeit

zumindest im ersten Durchlauf, also ausgehend von frischem Monomer ohne Recyclat, einen Wert zwischen 0,10 und 0,16 $(kmol/m^3) \cdot h$, bevorzugt 0,11 bis 0,15 $(kmol/m^3) \cdot h$, annimmt. Durch den als Folge nur mäßigen Monomer-bzw. Edukt-Umsatz von bevorzugt ca. 50 % bis 60 % der eingesetzten Substanzmenge im ersten und allen nachfolgenden Reaktionsdurchläufen wird die Bildung von Heptameren und höheren Oligomeren verringert, die die Produktverteilung unerwünscht verbreitern. Bereits der nach dem ersten Reaktionsdurchlauf abgetrennte Teil an Tetrameren und höheren Oligomeren entspricht in dieser bevorzugten Ausführung des Verfahrens direkt den Anforderungen an das Produkt für einen Einsatz im Schmierölsektor. Die Anzahl der Reaktionsdurchläufe beträgt bevorzugt bis zu sieben. In aller Regel ist die Durchführung weiterer Reaktionsdurchläufe wegen der hohen benötigten Katalysatorkonzentration nicht mehr sinnvoll.

Die Reaktion kann erfindungsgemäß sowohl in Substanz als auch in inerten Lösemitteln durchgeführt werden.

Geeignete Lösemittel sowie geeignete Katalysatoren stellen die in DE-OS 41 19 332 genannten Stoffe dar. Die Reaktionstemperatur beträgt zwischen 150 °C und 350 °C, bevorzugt zwischen 180 °C und 210 °C; der Reaktionsdruck ergibt sich aus dem sich bei der Reaktionstemperatur einstellenden Dampfdruck der Reaktanden. Bevorzugt werden als monomere $\alpha,\omega$-Diolefine 1,4-Pentadien, 1,7-Octadien, 1,9-Decadien, 1,11-Dodecadien oder 1,4,9-Decatrien rein oder als Gemisch eingesetzt.

Zusätzlich zum Recyclat und zum frischen Monomer können weitere Mono-oder Diolefine oder beides vor einem oder mehreren Durchläufen zudosiert werden. Durch den Zusatz von höher- (oder niedriger-)molekularen Monomeren kann durch Co-Oligomerisierung die Produktverteilung hinsichtlich der Molasse in weiten Grenzen beeinflußt werden, so daß das Produkt an unterschiedliche Anforderungen angepaßt werden kann. Gleiches kann durch die Zudosierung von verglichen mit dem Monomer (etwa) gleichmolekularen $\alpha$-Monoolefinen erreicht werden.

Das erfindungsgemäße Verfahren kann sowohl diskontinuierlich als auch kontinuierlich betrieben werden. Eine bevorzugte Ausführung der kontinuierlichen Reaktionsführung besteht darin, daß die verschiedenen Reaktionsdurchläufe oder -abschnitte in einem einzigen Reaktor unmittelbar aneinandergereiht werden, wobei sich etwa nach drei Durchläufen ein quasi-stationärer Zustand hinsichtlich der Edukte und des Produktaustrags jedes Durchlaufs, nicht jedoch bezüglich der benötigten Katalysatormenge einstellt. Dabei können die Übergänge zwischen den einzelnen Reaktionsdurchläufen derart verschwimmen, daß diese nur noch gedanklich zu trennen sind.

Das erfindungsgemäße Verfahren bietet die folgenden Vorteile:

- Es wird unabhängig vom eingesetzten monomeren $\alpha,\omega$-Diolefin stets eine enge Produktverteilung um den Oligomierungsgradbereich 4 bis 5 erreicht. Das Produkt weist damit verbesserte Eigenschaften für eine Verwendung als Grundöl für Schmierstoffe und funktionelle Flüssigkeiten auf.

- Die Produktverteilung und damit die Produkteigenschaften können durch Zudosierung von $\alpha$-Monoolefinen oder anderen $\alpha,\omega$-Diolefinen gezielt beeinflußt werden.

- Die Ausbeute an Tetra-, Penta- und Hexameren, die das eigentliche Wertprodukt darstellen, wird deutlich verbessert und damit die Wirtschaftlichkeit der Herstellung erhöht.

- Der Gehalt an Heptameren und höheren Oligomeren im Produkt wird verringert.

- Wegen der engen Produktverteilung kann eine destillative Aufarbeitung des Produktes entfallen. Die Summe der abgetrennten Teile, die die Tetramere und höheren Oligomere enthalten, kann direkt das Wertprodukt bilden.

Das erfindungsgemäße Verfahren wird durch die folgenden Beispiele näher erläutert:

Vergleichsbeispiel 1:

Gemäß DE-OS 41 19 332 werden 1 533 g 1,7-Octadien gelöst in 778 g Cyclohexan in einem Reaktor bei 200 °C mit Diisobutyl-Aluminium-Hydrid (DIBAH) als Katalysator umgesetzt. Nach einer Reaktionszeit von 2 Stunden hat das Reaktionsgemisch die in Tabelle 1 angegebene Zusammensetzung.
Der Gehalt an Tetra-, Penta- und Hexameren beträgt also etwa 30 Gew.-%.

Beispiel 1:

Das erfindungsgemäße Verfahren wird diskontinuierlich zur Umsetzung von 1,7-Octadien in einem Reaktor eingesetzt. Die Reaktionsbedingungen sowie die Edukt- und Produktzusammensetzung jedes Durchlaufs zeigen die Tabellen 2 und 3. Als Katalysator dient Diisobutyl-Aluminium-Hydrid (DIBAH). Die Reaktion wird in Cyclohexan als Lösemittel durchgeführt. Nach sieben Durchläufen wird das Verfahren abgebrochen. Durch Summenbildung über alle Durchläufe ergibt sich die in Tabelle 1 angegebene Produktzusammensetzung.
Man erkennt deutlich die höhere Ausbeute an Tetra-, Penta- und Hexameren sowie die engere Produktverteilung um den Oligomerisierungsgrad 4 bis 5 im Vergleich mit Vergleichsbeispiel 1.

Beispiel 2:

Das erfindungsgemäße Verfahren wird kontinuierlich, aber instationär zur Umsetzung von 1,9-Decadien (gelöst in Cyclohexan) in einem einzigen Strömungsrohr durchgeführt. Das Reaktionsvolumen beträgt 3,2 m$^3$ und die mittlere Verweilzeit je Durchlauf im Rohrreaktor ca. 2 h.
Beim Start werden zunächst als Edukt 0,08 kmol/min 1,9-Decadien gelöst in 9,5 kg/min Cyclohexan des Strömungsrohr zugeführt, da zu diesem Zeitpunkt noch kein Recyclat vorliegt. Als Katalysator werden 0,0016 kmol/min DIBAH beim Start zugesetzt. Sobald das erste Recyclat anfällt (ca. nach der mittleren Verweildauer von 2 h), wird die Zufuhr von frischem Monomer als Edukt im Zulauf soweit zurückgenommen, daß stets etwa dieselbe Menge an endständigen Doppelbindungen zum Einsatz kommt. Gleichzeitig wird die zugeführte Katalysatormenge erhöht, und zwar stufenweise ca. alle 2 h bis auf den Endwert von 0,0025 kmol/min im letzten (gedanklichen) Durchlauf. Etwa ab dem dritten Durchlauf, also nach ca. 6 h, arbeitet das Verfahren quasistationär hinsichtlich des Recyclats, der Edukte und des Produktaustrages; lediglich die Katalysatormenge variiert von Durchlauf zu Durchlauf. Als Recyclat werden dann ca. 70 Gew.-% des Gesamtaustrages an Leichtsiedern (Monomere bis Trimere des 1,9-Decadien) rückgeführt und erneut als Teil der Edukte eingesetzt. Die Reaktionskennzahl jedes Durchlaufs beträgt ca. 0,13 (kmol/m$^3$ ) · h.

Die Zusammensetzung des (Wert-)Produktes (nach der Abtrennung der Leichtsieder) in diesem quasistationären Zustand ist in Auszügen in Tabelle 1 angegeben. Der Anteil an Tetra-, Penta- und Hexameren im Wertprodukt beträgt ca. 68 Gew.-% und ist damit mehr als doppelt so hoch wie in Vergleichsbeispiel 1. Nach ca. 14 Stunden wird das Verfahren unterbrochen (dies entspricht ca. 7 Durchläufen) und mit frischem Monomeren neu gestartet.

Beispiel 3:

Analog zu Beispiel 2 wird das erfindungsgemäße Verfahren zur kontinuierlichen, instationären Umsetzung von 1,7-Octadien (gelöst in Cyclohexan) verwendet. Anstelle des Strömungsrohres dient eine Kesselkaskade bestehend aus 4 Kesseln mit einem Reaktionsvolumen von jeweils 0,75 m$^3$ als Reaktor. Die Aufarbeitung des Rohproduktes mit der Abtrennung der Leichtsieder erfolgt lediglich am Austritt aus der Kesselkaskade, nicht nach jedem Kessel. Die mittlere Verweilzeit beträgt ca. 0,5 h je Kessel. Hinsichtlich der Eduktmenge, des Lösemittelbedarfs, des Katalysatorbedarfs, des Rückführungsgrades für das Recyclat sowie der Reaktionskennzahl gelten wieder die Angaben wie in Beispiel 2.

Auch hier ergibt sich u. a. für den Produktaustrag nach ca. 6 h ein quasistationärer Zustand. Die Zusammensetzung des Wertproduktes (nach der Abtrennung der Leichtsieder) eines Durchlaufs zeigt wieder Tabelle 1. Auch hier ist der Anteil an Tetra-, Penta- oder Hexameren mit ca. 64 Gew.-% mehr als doppelt so hoch wie in Vergleichsbeispiel 1. Auch hier wird das Verfahren nach ca. 14 Stunden unterbrochen und mit frischem Monomeren neu angesetzt.

Sowohl in Beispiel 2 als auch in Beispiel 3 ist die deutlich engere Produktverteilung um den Oligomerisierungsgrad 4 bis 5 im Vergleich zu Vergleichsbeispiel 1 zu erkennen. Zwar wurde in Beispiel 2 und in Beispiel 3 nur die Zusammensetzung des Wertproduktes eines quasistationären Durchlaufes ach Abtrennung der Leichtsieder betrachtet. Natürlich ist eine solche Aufarbeitung auch bezüglich des Produktes nach Vergleichsbeispiel 1 möglich. Nach Abtrennung der Leichtsieder verbliebe dort jedoch der hohe Anteil an Heptameren und höheren Oligomeren im Produkt, der im Vergleich mit dem erfindungsgemäßen Verfahren die Produktverteilung deutlich verbreitert und die Ausbeute an Tetra-, Penta- und Hexameren verringert.

Tabelle 1

| Produktzusammensetzungen | | | | |
|---|---|---|---|---|
| Oligomere | Stand der Technik | erfindungsgemäß | | |
| | Vergleichsbeispiel 1 Gew.-% | Beispiel 1 Gew.-% | Beispiel 2 Gew.-% | Beispiel 3 Gew.-% |
| Monomere | 9,5 | 8,9 | vernachlässigbar | vernachlässigbar |
| Dimere | 6,9 | 8,8 | vernachlässigbar | vernachlässigbar |
| Trimere | 7,2 | 14,1 | 12,7 | 13,0 |
| Tetramere | 10,6 | 19,8 | 33,4 | 31,3 |
| Pentamere | 10,6 | 16,3 | 20,9 | 19,6 |
| Hexamere | 8,9 | 11,7 | 13,7 | 13,4 |
| Heptamere | 8,9 | 20,4 | 7,7 | 7,9 |
| Octamere | 6,5 | | 5,2 | 5,8 |
| Nonamere u. höhere | 30,9 | | Rest | Rest |

Tabelle 2: Beispiel 1 - Edukte der Durchläufe

| Vers.- | Edukte | | | | | | | | | Temp. | Reakt.- |
| Nr. | frisches | aus vorausgegangenem Durchlauf | | | | Masse | Ges. Stoffmenge | Kat. DIBAH | LM Cyclo-hexan | | Zeit |
| | Okt. 1,7 | Monomere | Dimere | Trimere | Sonstige | | | | | | |
| | [g] | [g] | [g] | [g] | [g] | [g] | [mol] | [mol] | [g] | [°C] | [min] |
| 1 | 880 | - | - | - | - | 880 | 8 | 0,16 | 952 | 200 | 120 |
| 2 | 511 | 252 | 172 | 92 | 2 | 1029 | 8 | 0,17 | 952 | 200 | 120 |
| 3 | 576 | 187 | 159 | 122 | 6 | 1050 | 8 | 0,19 | 952 | 200 | 120 |
| 4 | 467 | 265 | 226 | 115 | 10 | 1083 | 8 | 0,22 | 952 | 200 | 120 |
| 5 | 434 | 277 | 218 | 185 | 15 | 1129 | 8 | 0,25 | 952 | 200 | 120 |
| 6 | 446 | 255 | 226 | 202 | 11 | 1140 | 8 | 0,28 | 952 | 200 | 120 |
| 7 | 195 | 142 | 131 | 119 | 9 | 596 | 4 | 0,14 | 476 | 200 | 120 |

EP 0 731 070 B1

Tabelle 3: Beispiel 1 - Produkte der Durchläufe

| | Produkte | | | | | | | | | |
|---|---|---|---|---|---|---|---|---|---|---|
| Vers-. Nr. | Monomere [g] | Dimere [g] | Trimere [g] | n = 4 [g] | n = 5 [g] | n = 6 [g] | n ≥ 7 [g] | Sonstige [g] | Gesamtmasse [g] | % der einge- setzten Masse |
| 1 | 280 | 192 | 163 | 63 | 54 | 31 | 36 | 3 | 821 | 93 |
| 2 | 187 | 163 | 166 | 105 | 98 | 72 | 157 | 6 | 948 | 92 |
| 3 | 265 | 232 | 159 | 91 | 64 | 42 | 49 | 10 | 912 | 87 |
| 4 | 277 | 221 | 195 | 89 | 80 | 59 | 84 | 15 | 1020 | 94 |
| 5 | 258 | 248 | 230 | 92 | 74 | 46 | 58 | 11 | 1017 | 90 |
| 6 | 180 | 168 | 181 | 101 | 84 | 80 | 197 | 11 | 1002 | 88 |
| 7 | 190 | 164 | 152 | 35 | 20 | 10 | 14 | 14 | 599 | 100 |

(Das Formelzeichen n steht für den Oligomerisierungsgrad)

EP 0 731 070 B1

**Patentansprüche**

1.  Verfahren zur Herstellung eines Oligomer-Gemisches aus $\alpha,\omega$-Diolefinen in Gegenwart von aluminiumorganischen Verbindungen als Katalysator in flüssiger Phase, gekennzeichnet durch

    -   einen Verfahrensablauf, der sich zumindest gedanklich aus mindestens zwei nacheinander angeordneten Reaktionsdurchläufen zusammensetzt,
    -   das Trennen des Reaktionsproduktes jedes Durchlaufs in einen ersten Teil, der zumindest - bezogen auf den Gesamtgehalt im Reaktionsprodukt - jeweils mehr als 95 Gew.-% der monomeren $\alpha,\omega$-Diolefine und ihrer Dimere sowie mehr als 60 Gew.-% ihrer Trimere enthält, und zumindest einen zweiten Teil, der zumindest die Tetramere und höheren Oligomere enthält,
    -   das Einsetzen zumindest eines Teiles der abgetrennten monomeren $\alpha,\omega$-Diolefine, Dimere und Trimere aus dem ersten Teil als Teil der Edukte für den jeweils nachfolgenden Durchlauf und Reduzieren der Einsatzmenge an frischem Monomer, so daß die Summe der endständigen Doppelbindungen bei jedem Durchlauf nahezu konstant bleibt,
    -   das Erhöhen der Katalysatorkonzentration von Durchlauf zu Durchlauf um bis zu 20 Gew.-% bezogen auf die Katalysatormasse des vorangegangenen Durchlaufs,
    -   einen Wert für die Reaktionskennzahl

    $$\text{Katalysator-Konzentration} \cdot \text{Reaktionszeit}$$

    zwischen 0,10 $(\text{kmol/m}^3) \cdot \text{h}$ und 0,16 $(\text{kmol/m}^3) \cdot \text{h}$ zumindest für den ersten Durchlauf.

2.  Verfahren nach Anspruch 1,
    gekennzeichnet dadurch,

    -   daß die Reaktionskennzahl einen Wert zwischen 0,11 $(\text{kmol/m}^3) \cdot \text{h}$ und 0,15 $(\text{kmol/m}^3) \cdot \text{h}$ im ersten Durchlauf annimmt.

3.  Verfahren nach Anspruch 1 oder 2,
    gekennzeichnet dadurch,

    -   daß als Katalysator aluminiumorganische Verbindungen der Formel $AlX_3$ oder $AlX_2H$ verwendet werden, wobei X einen aliphatischen, alicyclischen oder aromatischen Kohlenwasserstoffrest mit 1 bis 30 Kohlenstoffatomen darstellt.

4.  Verfahren nach Anspruch 1, 2 oder 3,
    gekennzeichnet dadurch,

    -   daß die Katalysator-Konzentration im ersten Durchlauf zwischen 2 und 4 Mol-% bezogen auf die eingesetzte Substanzmenge an $\alpha,\omega$-Diolefinen beträgt.

5.  Verfahren nach einem der Ansprüche 1 bis 4,
    gekennzeichnet dadurch,

    -   daß die Reaktionstemperatur zwischen 180 °C und 210 °C beträgt und daß der Druck dem sich dabei einstellenden Dampfdruck der Reaktanden entspricht.

6.  Verfahren nach einem der Ansprüche 1 bis 5,
    gekennzeichnet dadurch,

    -   daß das Rohprodukt jedes Durchlaufs zunächst einer hydrolytischen Zerstörung des Katalysators unterzogen wird.

7.  Verfahren nach einem der Ansprüche 1 bis 6,
    gekennzeichnet dadurch,

    -   daß die Trennung in die im Anspruch 1 genannten Teile destillativ erfolgt.

**8.** Verfahren nach einem der Ansprüche 1 bis 7,
gekennzeichnet dadurch,

- daß nach jedem Durchlauf die Monomeren und Dimeren (nahezu) vollständig und die Trimeren zu mehr als 60 Gew.-% des Gesamtgehalts im Rohprodukt abgetrennt werden und
- daß in einem nachfolgenden Durchlauf jeweils 60 bis 80 Gew.-% dieser abgetrennten Monomere und Dimere sowie (nahezu) alle abgetrennten Trimere als Teil der Edukte zum Einsatz kommen.

**9.** Verfahren nach einem der Ansprüche 1 bis 8,
gekennzeichnet dadurch

- daß die Reaktion in Substanz oder in einem inerten Lösemittel durchgeführt wird.

**10.** Verfahren nach einem der Ansprüche 1 bis 9,
gekennzeichnet dadurch,

- daß das Verfahren kontinuierlich oder diskontinuierlich durchgeführt wird.

**11.** Verfahren nach einem der Ansprüche 1 bis 10,
gekennzeichnet dadurch,

- daß als monomere $\alpha,\omega$-Diolefine 1,4-Pentadien, 1,7-Octadien, 1,9-Decadien, 1,11-Dodecadien oder 1,4,9-Decatrien eingesetzt werden.

**12.** Verfahren nach einem der Ansprüche 1 bis 11,
gekennzeichnet dadurch,

- daß die mittlere Molmasse des Oligomer-Gemisches (also des Produktes) durch Zugabe von $\alpha$-Monoolefinen oder durch Co-Oligomerisierung mit $\alpha,\omega$-Diolefinen mit vom Hauptmonomeren abweichender Kohlenstoffkettenlänge gesteuert wird.

**13.** Verwendung der gemäß Anspruch 1 hergestellten Oligomer-Gemische für biologisch abbaubare Grundöle für Schmierstoffe und funktionelle Flüssigkeiten.

## Claims

**1.** A process for preparing an oligomer mixture from $\alpha,\omega$-diolefins in the presence of organoaluminium compounds as catalyst in the liquid phase, characterized by

- a process course which is composed, at least conceptually, of at least two successive reaction passes,
- the separation of the reaction product of each pass into a first part which contains at least, based on the total content in the reaction product, in each case more than 95% by weight of the monomeric $\alpha,\omega$-diolefins and their dimers and also more than 60% by weight of their trimers, and at least one second part which contains at least the tetramers and higher oligomers,
- the use of at least a part of the separated monomeric $\alpha,\omega$-diolefins, dimers and trimers from the first part as part of the starting materials for the subsequent pass in each case and reduction of the amount of fresh monomer used, so that the total of the terminal double bonds remains almost constant in each pass,
- increasing the catalyst concentration from pass to pass by up to 20% by weight based on the catalyst mass of the preceding pass,
- a value for the reaction characteristic

$$\text{catalyst concentration} \cdot \text{reaction time}$$

of from 0.10 $(\text{kmol/m}^3) \cdot$ h to 0.16 $(\text{kmol/m}^3) \cdot$ h at least for the first pass.

**2.** A process according to claim 1, characterized in that

- the reaction characteristic assumes a value of from 0.11 $(\text{kmol/m}^3) \cdot$ h to 0.15 $(\text{kmol/m}^3) \cdot$ h in the first pass.

3. A process according to claim 1 or 2, characterized in that

   - organoaluminium compounds of the formula $AlX_3$ or $AlX_2H$ are used as catalyst, where X is an aliphatic, alicyclic or aromatic hydrocarbon radical having from 1 to 30 carbon atoms.

4. A process according to claim 1, 2 or 3, characterized in that

   - the catalyst concentration in the first pass is from 2 to 4 mol% based on the molar amount of $\alpha,\omega$-diolefins used.

5. A process according to any one of claims 1 to 4, characterized in that

   - the reaction temperature is from 180°C to 210°C and the pressure corresponds to the autogenous vapour pressure of the reactants.

6. A process according to any one of claims 1 to 5, characterized in that

   - the crude product of each pass is first subjected to a hydrolytic decomposition of the catalyst.

7. A process according to any one of claims 1 to 6, characterized in that

   - the separation into the parts specified in claim 1 is carried out by distillation.

8. A process according to any one of claims 1 to 7, characterized in that

   - after each pass the monomers and dimers are separated off (almost) completely and the trimers are separated off to an extent of more than 60% by weight of the total content in the crude product and
   - in a subsequent pass from 60 to 80% by weight of each of these separated monomers and dimers and (almost) all separated trimers are used as part of the starting materials.

9. A process according to any one of claims 1 to 8, characterized in that

   - the reaction is carried out in bulk or in an inert solvent.

10. A process according to any one of claims 1 to 9, characterized in that

   - the process is carried out continuously or batchwise.

11. A process according to any one of claims 1 to 10, characterized in that

   - 1,4-pentadiene, 1,7-octadiene, 1,9-decadiene, 1,11-dodecadiene or 1,4,9-decatriene are used as monomeric $\alpha,\omega$-diolefins.

12. A process according to any one of claims 1 to 11, characterized in that

   - the mean molecular mass of the oligomer mixture (i.e. the product) is controlled by addition of $\alpha$-monoolefins or by co-oligomerization with $\alpha,\omega$-diolefins having a carbon chain length different from the main monomer.

13. The use of the oligomer mixtures prepared according to claim 1 for biodegradable base oils for lubricants and functional liquids.

**Revendications**

1. Procédé de fabrication d'un mélange d'oligomères à base de dioléfines-$\alpha$, $\omega$, en présence de dérivés organiques d'aluminium comme catalyseur en phase liquide,
   caractérisé par

   - un déroulement du procédé qui se compose, au moins par la pensée, de deux cycles de réaction, disposés l'un après l'autre,

- la séparation du produit de réaction de chaque cycle dans une première partie qui renferme au moins, rapporté à la teneur totale dans le produit de la réaction, à chaque fois plus de 95 % en poids de dioléfines-$\alpha$, $\omega$ monomères et de leurs dimères ainsi que plus de 60 % en poids de leurs trimères et au moins une deuxième partie qui renferme au moins les tétramères et les oligomères supérieurs,
- la mise en oeuvre d'au moins une partie des dioléfines-$\alpha$, $\omega$ monomères, dimères et trimères séparés de la première partie en tant que partie des éduits par cycle chaque fois en suivant et réduction de la quantité utilisée en monomère frais, de façon que la somme des doubles liaisons terminales demeure à peu près constante à chaque cycle,
- l'élévation de la concentration de catalyseurs de cycle en cycle de jusqu'à 20 % en poids, rapporté à la masse de catalyseurs des cycles précédents,
- une valeur pour le nombre de référence de la réaction

Concentration en catalyseur x temps de réaction

entre 0,10 (kmol/m$^3$) x h et 0,16 (kmol/m$^3$) x h au moins pour le premier cycle.

2. Procédé selon la revendication 1,
caractérisé en ce que

le nombre de référence de la réaction prend une valeur comprise entre 0,11 (kmol/m$^3$) x h et 0,15 (kmol/m$^3$) x h dans le premier cycle.

3. Procédé selon la revendication 1 ou la revendication 2,
caractérisé en ce que

comme catalyseur, on utilise des dérivés organiques de l'aluminium de formule AIX$_3$ ou AIX$_2$H, dans laquelle X représente un radical hydrocarboné aliphatique, alicyclique ou aromatique ayant de 1 à 30 atomes de carbone.

4. Procédé selon la revendication 1, 2 ou 3,
caractérisé en ce que

la concentration en catalyseur dans le premier cycle s'élève à, entre 2 et 4 % molaire, rapporté à la quantité de substance mise en jeu de dioléfines-$\alpha$, $\omega$.

5. Procédé selon l'une des revendications 1 à 4,
caractérisé en ce que

- la température de réaction s'élève à, entre 180 et 210°C,
- la pression correspond à la pression de vapeur des réactifs se réglant dans ce but.

6. Procédé selon l'une des revendications 1 à 5,
caractérisé en ce que

le produit brut de chaque cycle est soumis, en premier lieu, à une décomposition hydrolytique du catalyseur.

7. Procédé selon l'une des revendications 1 à 6,
caractérisé en ce que

la séparation des parties mentionnées dans la revendication 1 s'effectue par distillation.

8. Procédé selon l'une des revendications 1 à 7,
caractérisé en ce que

- après chaque cycle, les monomères et les dimères sont séparés (à peu près) complètement et les trimères, pour plus de 60 % en poids de la teneur totale dans le produit brut,
- dans le cycle suivant à chaque foie, 60 à 80 % en poids de ces monomères et dimères séparés ainsi que (à peu près) tous les trimères séparés, viennent à utilisation en tant que partie des éduits.

9. Procédé selon l'une des revendications 1 à 8,
   caractérisé par le fait que

   la réaction est effectuée en substance ou dans un solvant inerte.

10. Procédé selon l'une des revendications 1 à 9,
    caractérisé en ce que

    le procédé est exécuté en continu ou d'une manière discontinue.

11. Procédé selon l'une des revendications 1 à 10,
    caractérisé en ce que

    comme dioléfines-$\alpha$, $\omega$ monomères, on met en oeuvre le 1,4-pentadiène, le 1,7-octadiène, le 1,9-décadiène, le 1,11-dodécadiène ou le 1,4,9-décatriène.

12. Procédé selon l'une des revendications 1 à 11,
    caractérisé en ce que

    la masse molaire moyenne du mélange d'oligomère (aussi du produit) est contrôlée par addition de $\alpha$-mono-oléfines ou par co-oligomérisation avec des dioléfines-$\alpha$, $\omega$ avec des longueurs de chaîne carbonée qui s'écarte des monomères principaux.

13. Utilisation des mélanges d'oligomères produits selon la revendication 1, pour des huiles de base biodégradables pour des produits de graissage ou pour des liquides fonctionnels.